# EUROPÄISCHE PATENTSCHRIFT

(11) **EP 1 242 345 B1**
(45) Veröffentlichungstag und Bekanntmachung des Hinweises auf die Patenterteilung: **15.09.2004**
(21) Anmeldenummer: 00990689.2
(22) Anmeldetag: 08.12.2000
(51) Int. Cl.: C07C 29/70, C07C 29/80

(54) **VERFAHREN ZUR HERSTELLUNG VON ALKALIMETHYLATEN**
METHOD FOR PRODUCING ALKALI METHYLATES
PROCEDE DE PRODUCTION DE METHYLATES ALCALINS

(30) Priorität: 08.12.1999 DE 19959153
(43) Veröffentlichungstag der Anmeldung: 25.09.2002
(73) Patentinhaber: BASF AKTIENGESELLSCHAFT, 67056 Ludwigshafen (DE)
(72) Erfinder: GUTH, Josef, 67251 Freinsheim (DE); FRIEDRICH, Holger, 67240 Bobenheim-Roxheim (DE); STERZEL, Hans-Josef, 67125 Dannstadt-Schauernheim (DE); KAIBEL, Gerd, 68623 Lampertheim (DE); BURKART, Kirsten, 67069 Ludwigshafen (DE); HOFFMANN, Elke, 07749 Jena (DE)
(74) Vertreter: Bardehle, Heinz, Dipl.-Ing.
(86) Internationale Anmeldenummer: PCT/EP2000/012440
(87) Internationale Veröffentlichungsnummer: WO 2001/042178

(56) Entgegenhaltungen:
- EP-A- 0 299 577
- DE-C- 968 903

## Beschreibung

Die Erfindung betrifft ein Verfahren zur Herstellung von Alkalimethylaten aus wäßriger gegebenenfalls mit Methanol versetzter Alkalilauge und Methanol.

Die Herstellung von Alkoholaten aus Alkalilaugen und Alkoholen ist an sich bekannt.

Bereits die DE-C 968 903 beschreibt die kontinuierliche Behandlung hochprozentiger wäßriger Alkalihydroxidlösungen mit entgegengesetzt strömendem Alkohol in einer Füllkörperkolonne, wobei die Zufuhr der Alkalilauge an verschiedenen Stellen der Kolonne erfolgen kann, um das Verstopfen der Kolonne durch sich abzusetzendes Alkalihydroxid zu verhindern. Das Alkoholat bildet sich durch Reaktion des auf den Füllkörpern befindlichen Alkalihydroxides mit dem zusätzlich von oben nach unten geführtem Alkoholstrom.

Niedersiedende Alkohole (Methanol) lassen sich allerdings nach diesem Verfahren nur dann verwenden, wenn eine geeignete Hilfsflüssigkeit zum Auskreisen des Wassers eingesetzt wird. Dieses Verfahren hat ferner den Nachteil, daß die sich auf den Füllkörpern der Kolonne absetzenden Ablagerungen von festem Alkalihydroxid zu Verstopfungen der Kolonne führen können. Die in der DE-C 968 903 beschriebene Arbeitsweise erwähnt zwar das azeotrope Abdestillieren des Alkohols mit Hilfe eines Schleppmittels. Bei der dort geschilderten Arbeitsweise muß jedoch der Alkohol des abdestillierten Alkohol-Wasser-Azeotrops konstant erneuert werden, wozu eine aufwendige Rückgewinnung notwendig ist.

Die SU-A 165 691 führt ein Verfahren zur Herstellung von Natriummethylat aus, das von einer methanolischen Natriumhydroxidlösung ausgeht, die aus einem Rührbehälter in den oberen Teil einer kontinuierlich arbeitenden Füllkörperkolonne mit dampfförmigem Methanol in Gegenstrom geführt wird.

Das Verfahren erfordert einen hohen Energieaufwand zum Erreichen der entsprechenden Produkteinheit. Es weist desweiteren den Nachteil auf, daß festes Natriumhydroxid zur Anwendung kommt.

Die EP-A 0 299 577 beschreibt ein Verfahren zur Herstellung von Alkoholaten, bei dem die Abtrennung des Reaktionswassers durch Pervaporation erreicht wird. Dabei kann bekanntlich keine vollständige Entwässerung des Alkohols bewirkt werden.

Die DE-C 34 13 212 und DE-C 37 01 268 wiesen Verfahren zur Herstellung von Kalium-tert.-butoxid aus wäßriger Kalilauge und einem Überschuß an tert.-Butanol in einer Destillationskolonne unter Einsatz eines Schleppmittels aus, das im Gegensatz zu der in DE-C 968 903 beschriebenen Hilfsflüssigkeit kein Azeotrop mit Wasser bildet.

Darüber hinaus wird in RO-A 60485 die Gewinnung von Natriummethylat aus wasserfreiem technischem Methanol und technischem Natriumhydroxid in einem Dreistufenprozeß unter Zuhilfenahme eines Kohlenwasserstoffes, bei dem das entstehende Reaktionswasser durch Azeotropdestillation entfernt wird, beschrieben. Die Rückgewinnung wasserfreien Kohlenwasserstoffes erfolgt durch fraktionierte Destillation unter Zugabe von Na₂SO₄. Das Natriummethylat fällt in einer Kohlenwasserstoffsuspension an, von der es abfiltriert werden kann.

Diese Verfahren zeichnen sich aus, durch die Notwendigkeit zusätzlicher Trennoperationen, sowie durch den Verbleib von Fremdstoffen in der Alkalialkoholatlösung, die sich bei der Weiterverarbeitung störend bemerkbar machen.

Es hat sich experimentell herausgestellt, daß die theoretische Trennstufenzahl allein kein hinreichendes Kriterium für ein gut funktionierendes Verfahren zur Herstellung von weitgehend wasserfreien Alkoholatlösungen ist. So zeigen bekannte Verfahren zur Herstellung von Alkoholaten, daß selbst in Kolonnen, die eine hohe Trennstufenzahl von 35 bis 40 theoretischen Böden aufweisen, nur unbefriedigende Qualitäten erhalten werden können, bei denen der auf das Alkoholat bezogene Wassergehalt bei etwa 0,5 bis 2 % liegt. Diese Wassergehalte liegen oberhalb der Werte von marktüblichen Qualitäten mit einem Wassergehalt von etwa 0,1 %.

Der Erfindung liegt daher die Aufgabe zugrunde, ein Verfahren zur Herstellung von Alkoholatlösungen bereitzustellen, das es ermöglicht, mit vertretbarem Energieaufwand ein weitgehend wasserfreies Endprodukt zu erhalten, wobei beim Einsatz einer bepackten Reaktionskolonne keine Ablagerungen auf den Füllkörpern entstehen.

Diese Aufgabe wird erfindungsgemäß dadurch gelöst, daß das an sich bekannte Verfahren zur Herstellung von Alkalimethylaten aus wäßriger gegebenenfalls mit Methanol versetzter Alkalilauge und Methanol in einer Reaktionskolonne, mit mindestens 5, bevorzugt 15 bis 30 theoretischen Böden zwischen der Zulaufstelle der Alkalilauge und der Zugabestelle des Methanols durchgeführt wird, wobei für die als Glocken-, Ventil- oder Siebbödenkolonnen ausgebildete Reaktionskolonne Böden gewählt werden, bei denen maximal 5 % bevorzugt ≤ 1% der Flüssigkeit durch die jeweiligen Böden durchregnet.

Für diese Ausgestaltung des erfindungsgemäßen Verfahrens sind grundsätzlich Glockenböden-, Ventilböden- und Siebbödenkolonnen geeignet. Speziell für Ventilböden und Siebböden sind die Böden so auszuführen, daß das Durchregnen der Flüssigkeit minimiert wird. Die hierzu erforderlichen konstruktiven Maßnahmen sind dem Fachmann geläufig. Es werden besonders dicht schließende Ventilbauarten gewählt und damit insbesondere die Dampfgeschwindigkeit in den Bodenöffnungen auf bis zu dem doppelten Wert erhöht, der üblicherweise eingestellt wird. Dies wird durch eine Verringerung der Zahl der Ventile erreicht. Bei Siebböden ist es besonders günstig, die Durchmesser der Bodenöffnungen zu verringern und die Zahl der Öffnungen beizubehalten oder noch zu vergrößern.

In einer erfindungsgemäßen Variante ist die Reaktionskolonne mit Füllkörpern oder geordneten Packungen ausgestattet, wobei die geordneten Packungen im Hinblick auf die Gleichmäßigverteilung der Flüssigkeit Füllkörpern vorzuziehen sind. Bei dieser Ausführungsform der Erfindung darf ferner in allen Teilbereichen des Kolonnenquerschnitts, die mehr als 2 % des gesamten Kolonnenquerschnitts entsprechen, das gemittelte Verhältnis von Flüssigkeits-zu Dampfstrom hinsichtlich der Flüssigkeit um nicht mehr als 15 %, bevorzugt um nicht mehr als 3 % überschritten werden.
Diese erfindungsgemäß niedrig gehaltene Flüssigkeitsmenge macht offensichtlich möglich, daß der Kapillareffekt an den Drahtgeweben lokale Spitzenwerte der Flüssigkeitsberieselungsdichte ausschließt.

Geeignete Verfahren hierzu sind aus EP A 0 684 060 bekannt. Die gewünschte Charakteristik der Flüssigkeitsverteilung kann bei Anwendung von Füllkörperund Packungskolonnen dadurch erzielt werden, dass in dem dem Kolonnenmantel benachbarten Randbereich des Kolonnenquerschnitts, der etwa 2 bis 5 % des gesamten Kolonnenquerschnitts entspricht, die Flüssigkeitsberieselungsdichte gegenüber dem übrigen Querschnittsbereich um bis zu 100 %, bevorzugt um 5 bis 15 %, verringert wird. Dies lässt sich durch die gezielte Verteilung des Abtropfstellen der Flüssigkeitsverteiler oder deren Bohrungen mit einfachen Mitteln erreichen.

Bei dieser Arbeitsweise ist es zweckmäßig, daß die Kolonneninnenwand der Reaktionskolonne eine Temperatur aufweist, die 3 bis 10°C über der Temperatur der Reaktionskolonne liegt.

Offenbar muß wegen der sehr ungünstigen Lage des chemischen Gleichgewichts ein Durchschlagen auch kleinster Mengen an noch wasserhaltiger Flüssigkeit, die nicht mit einer ausreichend großen Brüdenmenge in Kontakt gekommen sind, mit Sicherheit ausgeschlossen werden. Lokal durchschlagende wasserhaltige Flüssigkeitsmengen können offensichtlich die Qualität des Produktes durch Rückreaktion entscheidend verschlechtern.

Die Kombination des an sich bekannten Verfahrens zur Herstellung von Alkalimethylaten mit den Merkmalen der Erfindung, die sicherstellen, daß an keiner Stelle des Reaktionskolonnenquerschnitts Randgängigkeiten oder Bachbildung beziehungsweise Durchregnen der Flüssigkeit eintreten, bewirkt in überraschender und synergistischer Weise einen Reinheitsgrad der Alkalimethylat-Lösung, wie dies bisher nur im Amalgamverfahren oder über die Verwendung von Alkalimetallen erzielbar war.

Es ist festzuhalten und für die technische Realisierung von Vorteil, daß hinsichtlich der Gleichförmigkeit der Gasströmung über die Querschnittsfläche der Kolonne keine zusätzliche konstruktive Forderungen zu stellen sind. Es ist nicht nachteilig, wenn das Verhältnis von Gas- zu Flüssigkeitsströmung lokal höhere Werte annimmt, als es dem Durchschnittswert entspricht. Durch erhöhte Gasströme wird die Wasserentfernung lokal eher verbessert.

Bei der Durchführung des erfindungsgemäßen Verfahrens erhält man keine Verstopfung in einer mit Füllkörpern oder geordneten Packungen versehenen Reaktionskolonne, und man kann das eingesetzte Methanol ohne aufwendiges Aufarbeiten dem Prozeß wieder zurückführen.

Das erfindungsgemäße Verfahren kann sowohl diskontinuierlich als auch kontinuierlich betrieben werden.

Beim diskontinuierlichem Betrieb erfolgt die Verdampfung des Alkohols und des Was sers bis zu dem Punkt, an dem die gewünschte Alkoholatkonzentration im Reaktions gemisch vorliegt.

Beim kontinuierlichen Verfahren wird am Kopf der Reaktionskolonne ein wässriger gegebenenfalls mit Methanol versetzter Alkalilaugestrom zugefahren. Die Reaktionsko lonne wird als reine Abtriebskolonne betrieben. Dem unteren Bereich der Kolonne wird dampfförmig Methanol zugefahren. Über den Sumpfabzug erhält man spezifikationsgerechtes Alkoholat. Der am Kolonnenkopf entweichende, Wasser enthaltende Methanolstrom wird in der Rektifikationskolonne aufgearbeitet. Am Kopf dieser Kolonne wird das benötigte Frisch-Methanol zugefahren. Dabei wird am Kolonnenkopf nach partieller Kondensation ein gasförmiger Methanolstrom mit sehr geringem Wasseranteil erhalten, der in den unteren Bereich der Reaktionskolonne zurückgeleitet wird.
Über den Sumpfaustrag der Rektifikationskolonne wird das Wasser aus dem System ausgeschleust.

Die bei dem erfindungsgemäßen Verfahren eingesetzte Alkalilauge wird zweckmäßigerweise über ein Membranverfahren gewonnen. Dadurch ist es möglich, Alkoholate herzustellen, die völlig quecksilberfrei sind. Es ist ferner hinsichtlich des Energieverbrauchs günstig, daß die Alkalilauge eine Konzentration von mindestens 30 % aufweist, bevorzugt bis zur Löslichkeitsgrenze aufkonzentriert wird, und, daß diese vor Eintritt in die Reaktionskolonne über einen Wärmetauscher bis nahe an die Siedetemperatur aufgeheizt wird.

Das Methanol wird in einer solchen Menge eingesetzt, daß es gleichzeitig als Lösungsmittel für das erhaltene Alkalimethylat dient. Diese Menge soll dabei so gewählt werden, daß im Sumpf der Reaktionskolonne die gewünschte Konzentration der Alkalimethylat-Lösung vorliegt, vorzugsweise eine 30%-ige Lösung.

Die genannte Bedingung für die einzusetzende Menge an Methanol wird dann erfüllt, wenn diese 10- bis 50-fach, bevorzugt 35- bis 40-fach des Massenanteils an Wasser beträgt, der mit der Natronlauge eingetragen wird beziehungsweise 10-bis 50-fach, bevorzugt 30- bis 40-fach des Massenanteils an Wasser beträgt, der mit der Kalilauge eingetragen wird.

Nach dem erfindungsgemäßen Verfahren wird die Reaktionskolonne ohne Rücklauf betrieben.

In der Rektifikationskolonne empfiehlt sich ein Rücklaufverhältnis von mindestens 0,5, bevorzugt 0,8 bis 1,4, wenn der am Kopf der Reaktionskolonne entnommene Brüden bevorzugt dampfförmig in die Rektifikationskolonne geleitet wird und das an deren Kopf gewonnene Methanol, mit einem Wassergehalt von 20 bis 100 ppm, bevorzugt dampfförmig über einen Partialkondensator geführt wird, um anschließend über einen Brüdenverdichter dem unteren Ende der Reaktionskolonne zugeführt zu werden.

In einer Variante des erfindungsgemäßen Verfahrens beträgt das Rücklaufverhältnis der Rektifikationskolonne mindestens 0,6, bevorzugt 0,8 bis 1,4.

In diesem Fall wird der am Kopf der Reaktionskolonne entnommene Brüden über einen der Rektifikationskolonne vorgeschalteten Brüdenverdichter geleitet und das sodann in der Rektifikationskolonne gewonnene Methanol, mit einem Wassergehalt von 20 bis 100 ppm, bevorzugt dampfförmig über einen Partialkondensator dem unteren Ende der Reaktionskolonne zugeführt.

Die Abmessungen der Reaktionskolonne lassen sich im unteren Bereich der Kolonne verringern, wenn ein Teil des Methanols am oberen Ende oder im Bereich des oberen Endes der Reaktionskolonne dampfförmig zugegeben wird.

Bei dieser erfindungsgemäßen Arbeitsweise wird der Methanolstrom nur in einer Teilmenge von 10 bis 70 %, bevorzugt 30 bis 50 %, am unteren Ende der Reaktionskolonne eingespeist und die restliche Teilmenge in einem Einzelstrom oder auf mehrere Teilströme verteilt bevorzugt 1 bis 10 theoretische Böden, besonders bevorzugt 1 bis 3 theoretische Böden unterhalb der Zulaufstelle von Alkalilauge dampfförmig zugegeben.

Die Abmessungen der Reaktionskolonne lassen sich im unteren Bereich der Kolonne durch die wahlweise Anordnung von einem oder mehreren Zwischenverdampfem im oberen Bereich der Reaktionskolonne verringern. Bei dieser Gestaltung ist es auch möglich, Teilströme des Methanols in flüssiger Form im oberen Bereich der Reaktionskolonne zuzugeben.

Es ist vorteilhaft, die Reaktionskolonne bei einem Druck von 0,5 bis 40 bar, bevorzugt 1 bis 15 bar, besonders bevorzugt 3 bis 10 bar zu betreiben, da sich bei höherem Druck kleinere Heizleistungen und kleinere Methanolmengen realisieren lassen.

Die Wahl des Drucks in der Rektifikationskolonne kann in einem weiten Bereich frei getroffen werden. Es empfiehlt sich, bei etwa 1 bis 4 bar zu arbeiten. Im Falle einer Brüdenverdichtung wird das Druckgefälle zwischen den beiden Kolonnen zweckmäßigerweise so gewählt, daß die Brüdenverdichtung für das Methanol-Wasser-Gemisch oder alternativ den Methanolstrom mit geringem Aufwand möglich ist.

Das für die Reaktion und die Verdünnung der Alkalimethylat-Lösung benötigte Methanol wird bei Temperaturen bis hin zum Siedepunkt, bevorzugt bei Raum-temperatur, auf den Kopf der Rektifikationskolonne aufgegeben.

In einer weiteren, hinsichtlich der Investitionskosten vorteilhaften Ausführungs-form der Erfindung, sind die Reaktions- und Rektifikationskolonne in einem einzigen Kolonnenmantel untergebracht, bei dem der untere Bereich der Kolonne durch eine vertikale zwischen den Kolonnenwänden angebrachte Trennwand unterteilt ist, die eine Quervermischung von Flüssigkeits- und Brüdenströmen in diesem Teilbereich der Kolonne verhindert, beide Teile dieses längsunterteilten Bereichs einen eigenen Verdampfer aufweisen, über den man die Alkoholatlösung beziehungsweise das Wasser entnimmt, daß man das am Kopf der Kolonne bei einem Rücklaufverhältnis von mindestens 0,5, bevorzugt 0,8 bis 1,4 gewonnene Methanol, mit einem Wassergehalt von 20 bis 100 ppm, über einen Partialkondensator und Brüdenverdichter leitet und sodann am unteren Ende der Kolonne in dem Teilbereich zugibt, aus dem man die Alkoholatlösung abzieht.

Bei dieser Arbeitsweise erweist es sich als günstig, die Kolonne bei einem Druck von 0,5 bis 10 bar, bevorzugt 1 bis 8 bar, besonders bevorzugt 3 bis 5 bar zu betreiben.

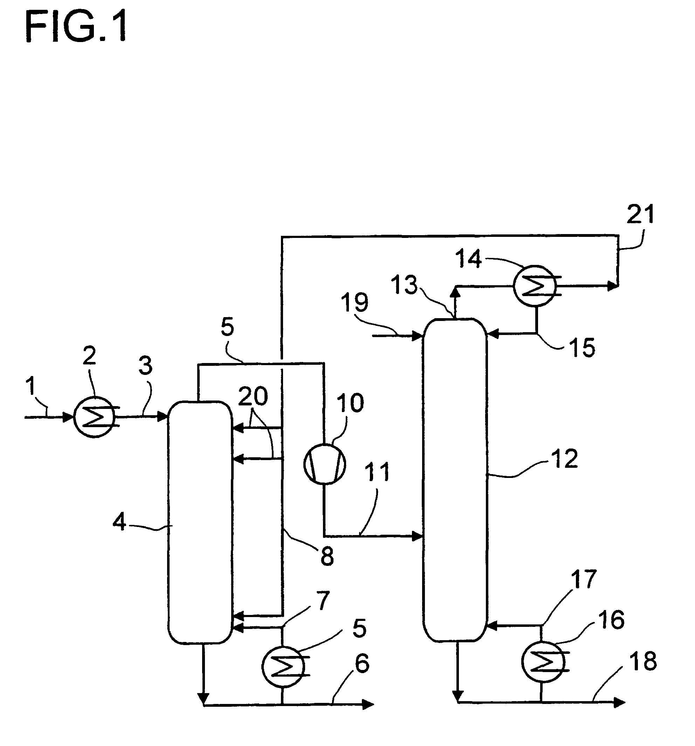

Das erfindungsgemäße Verfahren kann vorteilhaft in einer Apparatur gemäß den Abbildungen 1 bis 3 durchgeführt werden.

Abbildung 1 zeigt eine Apparatur, bei der die wäßrige Alkalilauge über die Rohrleitung 1 in einen Wärmetauscher 2 geführt wird, in der sie bis auf die Temperatur der Zulaufstelle aufgeheizt, gegebenenfalls teilverdampft und über die Rohrleitung 3 am Kopf der Kolonne 4 zugegeben wird. Die Alkalimethylatlösung wird am Sumpf der Kolonne über die Rohrleitung 6 entnommen. Am Sumpf der Kolonne befindet sich ein Verdampfer 5, mit dem die Konzentration der Alkalimethylatlösung auf den gewünschten Wert eingestellt wird. Der Brüden aus dem Verdampfer wird über die Rohrleitung 7 am unteren Ende der Kolonne zugegeben. Ebenfalls am unteren Ende der Kolonne wird dampfförmig Methanol über die Rohrleitung 8 zugefahren. Zusätzlich zu der Einspeisung über die Rohrleitung 8 kann dampfförmiges Methanol im oberen Bereich der Kolonne über eine oder mehrere Rohrleitungen 20 zugegeben werden. Am Kopf der Kolonne wird ohne Kondensation ein Methanol und Wasser enthaltender Strom 9 dampfförmig entnommen, über einen Brüdenverdichter 10 verdichtet und über die Rohrleitung 11 in die Destillationskolonne 12 zugefahren. Am Sumpf dieser Kolonne wird Wasser über die Rohrleitung 18 entnommen. Die Kolonne wird über den Verdampfer 16 beheizt, der den Brüdenstrom über die Rohrleitung 17 am unteren Ende der Kolonne zuführt. Am Kopf der Kolonne wird über die Rohrleitung 19 Methanol flüssig auf den Kopf der Kolonne aufgegeben. Der am Kopf der Kolonne anfallende Brüdenstrom wird über die Rohrleitung 13 in den Partialkondensator 14 geleitet, in dem ein Teil des Brüdens kondensiert und über die Rohrleitung 15 flüssig auf den Kopf der Kolonne aufgegeben wird und der restliche Brüdenstrom über die Rohrleitung 21 abgeführt und über die Rohrleitungen 8 und 20 in die Reaktionskolonne 4 gegeben wird.

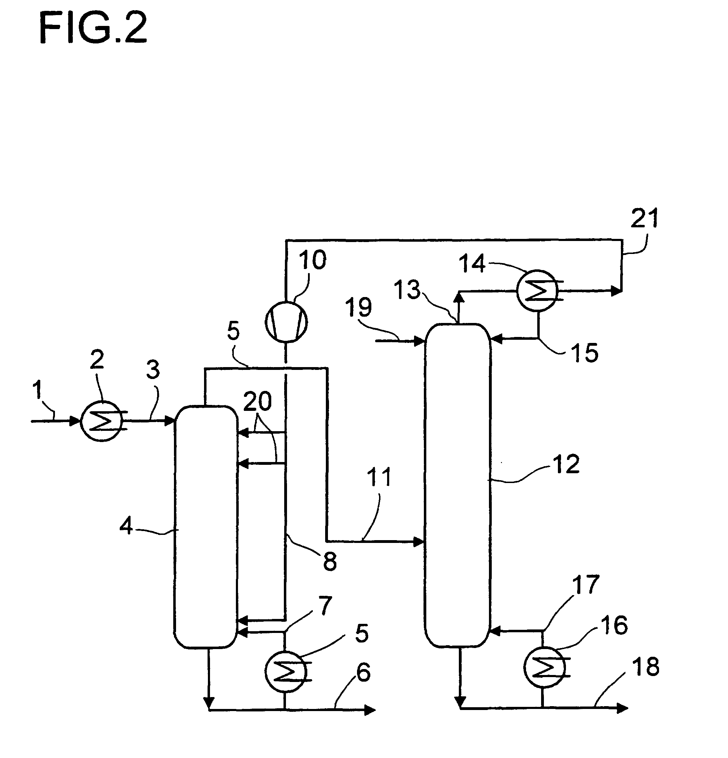

Abbildung 2 zeigt eine Variante des Ausführungsbeispiels 1, bei der die Reaktionskolonne 4 unter einem höheren Druck betrieben wird als die Kolonne 12. Der Brüdenstrom 9, der am Kopf der Kolonne 4 entnommen wird, wird direkt in die Kolonne 12 eingespeist. Der aus dem Partialkondensator 14 der Kolonne 12 entnommene Brüdenstrom 21 wird über den Verdichter 10 geschickt und von dort aus über die Rohrleitungen 8 und 20 der Kolonne 4 zugeführt.

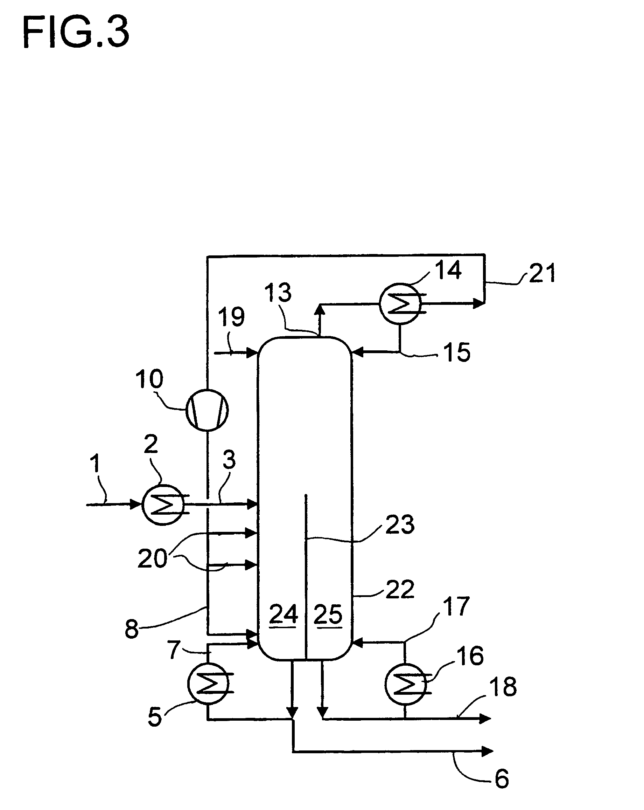

Abbildung 3 zeigt eine vorteilhafte apparative Vereinfachung, bei der die in den Abb. 1 und 2 gezeigten Kolonnen 4 und 12 zu einer einzigen Kolonne 22 zusammengefaßt sind. Diese Kolonne weist im unteren Bereich eine Trennwand 23 auf, die diesen Bereich in zwei Teilbereiche 24 und 25 aufteilt. Die wäßrige Alkalilauge wird über die Rohrleitung 1 in einen Wärmetauscher 2 geführt, in der sie bis auf die Temperatur der Zulaufstelle aufgeheizt, gegebenenfalls teilverdampft und über die Rohrleitung 3 am oberen Ende der Trennwand auf den Teilbereich 24 der Kolonne zugegeben wird. Am unteren Ende des Teilbereichs 24 der Kolonne befindet sich ein Verdampfer 5, mit dem die Konzentration der Alkalimethanolatlösung auf den gewünschten Wert eingestellt und die Alkalimethanolatlösung über die Rohrleitung 6 entnommen wird. Der Brüden aus dem Verdampfer wird über die Rohrleitung 7 am unteren Ende des Teilbereichs 24 der Kolonne zugegeben. Ebenfalls am unteren Ende des Teilbereichs 24 der Kolonne wird dampfförmig Methanol über die Rohrleitung 8 zugefahren. Zusätzlich zu der Einspeisung über die Rohrleitung 8 kann dampfförmiges Methanol im oberen Teil des Teilbereichs 24 der Kolonne über eine oder mehrere Rohrleitungen 20 zugegeben werden.

Am unteren Ende des Teilbereichs 25 der Kolonne wird Wasser über die Rohrleitung 18 entnommen.

Der Teilbereich 25 der Kolonne wird über den Verdampfer 16 beheizt, der den Brüdenstrom über die Rohrleitung 17 am unteren Ende des Teilbereichs 25 der Kolonne zuführt. Am Kopf der Kolonne wird über die Rohrleitung 19 Methanol flüssig auf den Kopf der Kolonne aufgegeben. Der am Kopf der Kolonne anfallende Brüdenstrom wird über die Rohrleitung 13 in den Partialkondensator 14 geleitet, in dem ein Teil des Brüdens kondensiert und über die Rohrleitung 15 flüssig auf den Kopf der Kolonne aufgegeben wird und der restliche Brüdenstrom über die Rohrleitung 21 abgeführt, im Brüdenverdichter 10 verdichtet und über die Rohrleitungen 8 und 20 in den Teilbereich 24 der Kolonne gegeben wird.

### Ausführungsbeispiele

### Beispiel 1

Ein auf 75°C erhitzter Stoffstrom von 27 g/h, bestehend aus 50%iger Natronlauge, wird am Kopf einer 1 m hohen, mit 3x3 mm Maschendrahtringen bestückten Kolonne zugefahren. Diese Schütthöhe entspricht ca. 20 theoretischen Stufen. Die Kolonne ist mit einem Doppelmantel ausgestattet, der in vier Kammern quer zur Kolonne unterteilt ist. In jede Kammer wird ein auf 80°C aufgeheizter Ölstrom gefahren. Die Kolonneninnentemperatur beträgt 71 bis 75°C. Die Kolonne wird ohne Rücklauf betrieben. Bei dieser Fahrweise sowie der Schutzbeheizung wird das gemittelte Verhältnis von Flüssigkeits- zu Dampfstrom hinsichtlich der Flüssigkeit um nicht mehr als 15 % überschritten und Randgängigkeit durch Verdampfung minimiert.

Die Kolonne wird bei Umgebungsdruck betrieben. Am unteren Ende der Schüttung werden 532 g/h eines flüssigen, ca. 20 ppm Wasser enthaltenden Methanolstroms mit einer Temperatur von 61°C zugefahren. Der Produktstrom aus dem Kolonnensumpf (61 g/h) besteht aus 30 Gew.-% Natriummethylat in Methanol und enthält ca. 60 ppm Wasser und 410 ppm Natriumhydroxid. Die Temperatur am Kolonnenkopf beträgt 75°C. Der Kopfabzug besteht aus Methanol mit einem Wasseranteil von 3,93 Gew.-% und entspricht einem Massenstrom von 498 g/h. Dieser Strom wird gasförmig in eine zweite Kolonne in Höhe der 5. theoretischen Stufe gefahren, die aus 40 Glockenböden, entsprechend 29 theoretischen Stufen, besteht und bei Normaldruck betrieben wird. Das Rücklaufverhältnis beträgt 1,3. Der nach der Totalkondensation am Kolonnenkopf entnommene Strom besteht aus ca. 30 ppm Wasser in Methanol und beträgt 478 g/h. Im Kondensatpuffer wird eine der Menge des aus der Anlage herausgefahrenen Methanolstroms entsprechende Menge Methanol (54 g/h) zugefahren. Der aus dem Kondensatbehälter abgezogene Strom wird in die Reaktionskolonne gefahren. Über den Sumpfabzug (20 g/h) der zweiten Kolonne wird das Wasser aus der Natronlauge und das bei der Reaktion entstandene Wasser mit einem Restgehalt von 1 Gew.-% Methanol aus dem System herausgefahren.

### Vergleichsbeispiel V2 (nicht erfindungsgemäß)

Ein auf 75°C erhitzter Stoffstrom von 29 g/h, bestehend aus 50 %iger Natronlauge, wird am Kopf einer 1 m hohen, mit 3x3 mm Maschendrahtringen bestückten Kolonne zugefahren. Diese Schütthöhe entspricht ca. 20 theoretischen Stufen. Die Kolonne ist mit einem Doppelmantel ausgestattet, der in vier Kammern quer zur Kolonne unterteilt ist. Die Kammern werden nicht mit Heizmedium beaufschlagt. Es wurde an der Kolonnenwandung eine Randgängigkeit der Flüssigkeit beobachtet. Dieser Flüssigkeitsstrom betrug ca. 3 bis 5 % bezogen auf den Gesamtflüssigkeitsstrom. Die Kolonne wird bei Umgebungsdruck betrieben. Am unteren Ende der Schüttung werden 535 g/h eines flüssigen, ca. 20 ppm Wasser enthaltenden Methanolstroms mit einer Temperatur von 60°C zugefahren. Der Produktstrom aus dem Kolonnensumpf (66 g/h) besteht aus 30 Gew.-% Natriummethylat in Methanol und enthält ca. 1070 ppm Wasser und 4060 ppm Natriumhydroxid. Die Kolonne wird ohne Rücklauf betrieben, und die Temperatur am Kolonnenkopf beträgt 75°C. Der Kopfabzug besteht aus Methanol mit einem Wasseranteil von 4,2 Gew.-% und entspricht einem Massenstrom von 498 g/h. Dieser Strom wird gasförmig in eine zweite Kolonne in Höhe der 5. theoretischen Stufe gefahren, die aus 40 Glockenböden, entsprechend 29 theoretischen Stufen, besteht und bei Normaldruck betrieben wird. Das Rücklaufverhältnis beträgt 1,3. Der nach der Totalkondensation am Kolonnenkopf entnommene Strom besteht aus ca. 30 ppm Wasser in Methanol und beträgt 477 g/h. Im Kondensatpuffer wird eine der Menge des aus der Anlage herausgefahrenen Methanolstroms entsprechende Menge Methanol (58 g/h) zugefahren. Der aus dem Kondensatbehälter abgezogene Strom wird in die Reaktionskolonne gefahren. Über den Sumpfabzug (20 g/h) der zweiten Kolonne wird das Wasser aus der Natronlauge und das bei der Reaktion entstandene Wasser mit einem Restgehalt von ca. 1 Gew.-% Methanol aus dem System herausgefahren.

### Beispiel 3

In Beispiel 3 wird die Natriummethylatsynthese in einer Trennwandkolonne wie sie in Abbildung 3 gezeigt wird, beschrieben. Bei kleinen Kolonnendurchmessern wie sie im Labor vorkommen, ist es einfacher, den Bereich der Trennwand durch zwei Kolonnen zu realisieren als den entsprechenden Kolonnenteil zu teilen. Deshalb wurde die Aufarbeitung mittels Trennwandkolonne im Abtriebsteil für den Laborversuch in der nachfolgend beschriebenen Weise aufgebaut: Der Teil 24 bestand aus einer 1 m langen Schüttung aus 3x3 mm Maschendrahtringen (20 theoretische Stufen), der Teil 25 aus einem Kolonnenschuss mit 10 Glocken (7 theoretische Stufen) und der Verstärkungsteil bestand ebenfalls aus einer Schüttung aus 3x3 mm Maschendrahtringen mit 1 m Länge (20 theoretische Stufen).
Der Kolonnenteil 24 war mit einem Doppelmantel ausgestattet, der in vier Kammern quer zur Kolonne unterteilt war. In jede Kammer wurde ein auf 80°C aufgeheizter Ölstrom gefahren. Die Kolonneninnentemperatur betrug 71 bis 75°C Somit wird Randgängigkeit durch Verdampfung vermieden.

Der Zulaufstrom von 20 g/h bestand aus 75°C heißer 50%igen Natronlauge. Dieser Strom wurde in Höhe der 20. theoretischen Stufe der Schüttung aus Maschendrahtringen, dh. am oberen Ende des Teils 24 in die Kolonne gefahren. Der Produktstrom (45 g/h) wurde aus dem Sumpfkreislauf des Teils 24 herausgefahren. Er bestand aus Natriummethylat in einem 69,5 %igen Methanolstrom mit 460 ppm Natriumhydroxid und ca. 65 ppm Wasser. Die Sumpftemperatur lag bei 71°C

Die Berieselungsdichte lag im Bereich des Doppelmantels in seiner Gesamtlänge bei 0,007 l/cm²*h. Diese niedrige Berieselung stellt sicher, daß durch den Kapillareffekt in der Schüttung das Verhältnis zwischen Flüssigkeits- und Dampfstrom sehr konstant über den Kolonnenquerschnitt ist und größere Abweichungen als 15 % vom Mittelwert dieses Verhältnisses nicht auftreten.

Der von der obersten Stufe der Maschendrahtringschüttung entweichende Dampfstrom gelangte in den gemeinsamen Verstärkungsteil. Die Kolonne wurde bei Normaldruck betrieben. Das Rücklaufverhältnis betrug 1,4. Am Kolonnenkopf wurde nach Totalkondensation und Ergänzung des aus dem System herausgefahrenen Methanols in den Kondensatauffangbehälter (40 g/h) ein ca. 25 ppm Wasser enthaltender Methanolstrom (50 g/h) erhalten, der in Höhe des unteren Randes der Schüttung wieder in den Kolonnenteil 24 eingespeist wurde. Am unteren Ende des Verstärkungsteils befand sich ein Rücklaufteiler, der so eingestellt war, daß der gesamte abfließende Strom in den Kolonnenteil 25 geleitet wurde. Der aus dem Sumpfkreislauf von Teil 25 herausgefahrene Strom enthielt noch ca. 1 Gew.-% Methanol und betrug 15 g/h.

## Patentansprüche

1. Verfahren zur Herstellung von Alkalimethylaten aus wäßriger gegebenenfalls mit Methanol versetzter Alkalilauge und Methanol in einer Reaktionskolonne (4), mit mindestens 5 theoretischen Böden zwischen der Zulaufstelle der Alkalilauge (3) und der Zugabestelle des Methanols (8) und Zerlegung des bei der Reaktion gebildeten dampfförmigen Methanol-Wasser-Gemisches in einer Rektifikationskolonne (12), **dadurch gekennzeichnet, daß** für die als Glocken-, Ventil- oder Siebbödenkolonne ausgebildete Reaktionskolonne (4) Böden gewählt werden, bei denen maximal 5 % der Flüssigkeit durch die jeweiligen Böden durchregnet, oder daß die Reaktionskolonne (4) mit Füllkörpern oder geordneten Packungen ausgestattet ist, bei der in allen Teilbereichen des Kolonnenquerschnitts, die mehr als 2 % des gesamten Kolonnenquerschnitts entsprechen, das gemittelte Verhältnis von Flüssigkeits- zu Dampfstrom hinsichtlich der Flüssigkeit um nicht mehr als 15 % überschritten wird, indem in dem dem Kolonnenmantel benachbarten Randbereich des Kolonnenquerschnitts, der etwa 2 bis 5 % des gesamten Kolonnenquerschnitts entspricht, die Flüssigkeitsberieselungsdichte gegenüber dem übrigen Querschnittsbereich um bis zu 100 % verringert wird, und die Kolonneninnenwand der mit Füllkörpern oder geordneten Packungen ausgestatteten Reaktionskolonnen (4), (24) eine Temperatur aufweist, die 3 bis 10°C über der Innentemperatur der Reaktionskolonne liegt, daß die Reaktionskolonne ohne Rücklauf betrieben wird, und daß die Reaktions- und die Rektifikationskolonne entweder einzelne Kolonnen (4) und (12) sind oder in einem einzigen Kolonnenmantel (22) untergebracht sind.

2. Verfahren nach Anspruch 1, **dadurch gekennzeichnet, daß** man die Alkalilauge elektrochemisch, bevorzugt über ein Membranverfahren, herstellt.

3. Verfahren nach Anspruch 1 oder 2, **dadurch gekennzeichnet, daß** die Alkalilauge eine Konzentration von mindestens 30 % aufweist und daß man diese vor Eintritt in die Reaktionskolonne (4), (24) über einen Wärmetauscher (2) bis nahe an die Siedetemperatur aufheizt, die dem in der Reaktionskolonne vorliegenden Druck entspricht.

4. Verfahren nach einem der Ansprüche 1 bis 3, **dadurch gekennzeichnet, daß** der Massenanteil an Methanol 10- bis 50-fach des Massenanteils an Wasser beträgt, der mit der Alkalilauge eingetragen wird, wenn die Alkalilauge Natronlauge oder Kalilauge ist.

5. Verfahren nach einem der Ansprüche 1 bis 4, **dadurch gekennzeichnet, daß** man das in der Rektifikationskolonne (12), (25) bei einem Rücklaufverhältnis von mindestens 0,5 aus dem Methanol-Wasser-Gemisch gebildete Methanol, mit einem Wassergehalt von 20 bis 100 ppm, über einen Partialkondensator (14) und Brüdenverdichter (10) in das untere Ende der Reaktionskolonne (8), zurückführt.

6. Verfahren nach Anspruch 5, **dadurch gekennzeichnet, daß** man den nach der Rektifikation gewonnenen Methanolstrom mit einem Wassergehalt von 20 bis 100 ppm nur in einer Teilmenge von 10 bis 70 % am unteren Ende der Reaktionskolonne (8) einspeist und die restliche Teilmenge in einem Einzelstrom oder auf mehrere Teilströme (20) verteilt 1 bis 10 theoretische Böden unterhalb der Zulaufstelle der Alkalilauge (3) dampfförmig zugibt.

7. Verfahren nach einem der Ansprüche 1 bis 6, **dadurch gekennzeichnet, daß** man frisches Methanol bei Temperaturen bis hin zum Siedepunkt auf den Kopf der Rektifikationskolonne (19) aufgibt.

8. Verfahren nach Anspruch 1 **dadurch gekennzeichnet, daß** die Reaktions- (4) und die Rektifikationskolonne (12) einzelne Kolonnen sind.

9. Verfahren nach Anspruch 8, **dadurch gekennzeichnet, daß** man das in der Rektifikationskolonne (12) mit vorgeschaltetem Brüdenverdichter (10) bei einem Rücklaufverhältnis von mindestens 0,5 aus dem Methanol-Wasser-Gemisch gebildete Methanol mit einem Wassergehalt von 20 bis 100 ppm über einen Partialkondensator (14) in die Reaktionskolonne (4) zurückführt.

10. Verfahren nach Anspruch 8, **dadurch gekennzeichnet, daß** man die Reaktionskolonne (4) bei einem Druck von 0,5 bis 40 bar betreibt.

11. Verfahren nach Anspruch 1 **dadurch gekennzeichnet, daß** die Reaktions- und die Rektifikationskolonne in einem einzigen Kolonnenmantel (22) untergebracht sind, bei dem der untere Bereich der Kolonne durch eine vertikale zwischen den Kolonnenwänden angebrachte Trennwand (23) unterteilt ist, die eine Quervermischung von Flüssigkeits- und Brüdenströmen in diesem Teilbereich der Kolonne verhindert, beide Teile dieses längsunterteilten Bereichs einen eigenen Verdampfer (5), (16) aufweisen, über die man die Alkoholatlösung (6) sowie das Wasser (18) entnimmt.

12. Verfahren nach Anspruch 11, **dadurch gekennzeichnet, daß** man die Alkalilauge mit einer Konzentration von mindestens 30% aufgeheizt bis nahe an die Siedetemperatur, am oberen Ende der Trennwand (23)zugibt (3), aus dem man die Alkoholatlösung entnimmt (24).

13. Verfahren nach Anspruch 11, **dadurch gekennzeichnet, daß** man die Kolonne (22) bei einem Druck von 0,5 bis 10 bar betreibt.

## Claims

1. A process for preparing alkali metal methoxides from aqueous alkali metal hydroxide, which may have been admixed with methanol, and methanol in a reaction column (4) having at least 5 theoretical plates between the feed point for the aqueous alkali metal hydroxide (3) and the feed point for the methanol (8) with fractionation of the gaseous methanol/water mixture formed in the reaction in a rectification column (12), wherein the trays selected for the reaction column (4) configured as a bubble cap tray, valve tray or sieve tray column are trays in which not more than 5% of the liquid rains through the respective trays, or the reaction column (4) is provided with random packing or ordered packing and the average ratio of liquid flow to vapor flow is not exceeded by more than 15% in all subregions of the column cross section which correspond to more than 2% of the total column cross section by the liquid trickle density in the marginal region of the column cross section next to the column wall, which region corresponds to about 2-5% of the total column cross section, being reduced by up to 100% compared to the remaining cross section, and the internal column wall of the reaction column (4), (24) provided with random packing or ordered packing being at a temperature which is from 3 to 10°C above the internal temperature of the reaction column, the reaction column is operated without reflux, and the reaction column and the rectification column are either individual columns (4) and (12) or are accommodated within a single outer column wall (22).

2. A process as claimed in claim 1, wherein the aqueous alkali metal hydroxide is prepared electrochemically, preferably by means of a membrane process.

3. A process as claimed in claim 1 or 2, wherein the aqueous alkali metal hydroxide has a concentration of at least 30%, and is heated by means of a heat exchanger (2) to close to the boiling point at the pressure prevailing in the reaction column prior to entering the reaction column (4), (24).

4. A process as claimed in any of claims 1 to 3, wherein the mass of methanol is from 10 to 50 times the mass of water which is introduced with the aqueous alkali metal hydroxide when the alkali metal hydroxide is sodium hydroxide or potassium hydroxide.

5. A process as claimed in any of claims 1 to 4, wherein the methanol having a water content of from 20 to 100 ppm formed from the methanol/water mixture in the rectification column (12), (25) at a reflux ratio of at least 0.5 is recirculated via a partial condenser (14) and vapor compressor (10) to the lower end of the reaction column (8).

6. A process as claimed in claim 5, wherein only part, namely from 10 to 70%, of the methanol stream obtained after the rectification, which has a water content of from 20 to 100 ppm, is fed in at the lower end of the reaction column (8) and the remaining part is introduced, as a single stream or divided into a plurality of substreams (20), in gaseous form from 1 to 10 theoretical plates below the feed point for the aqueous alkali metal hydroxide (3).

7. A process as claimed in any of claims 1 to 6, wherein fresh methanol at temperatures up to the boiling point is fed in at the top of the rectification column (19).

8. A process as claimed in claim 1, wherein the reaction column (4) and the rectification column (12) are individual columns.

9. A process as claimed in claim 8, wherein the methanol having a water content of from 20 to 100 ppm formed from the methanol/water mixture in the rectification column (12) with upstream vapor compressor (10) at a reflux ratio of at least 0.5 is recirculated via a partial condenser (14) to the reaction column (4).

10. A process as claimed in claim 8, wherein the reaction column (4) is operated at a pressure of from 0.5 to 40 bar.

11. A process as claimed in claim 1, wherein the reaction column and the rectification column are accommodated within a single outer column wall (22) in which the lower region of the column is divided by a vertical dividing wall (23) installed between the column walls so as to prevent cross-mixing of liquid and vapor streams in this subregion of the column, both parts of this longitudinally divided region have their own vaporizer (5), (16) via which the alkoxide solution (6) or the water (18) is taken off.

12. A process as claimed in claim 11, wherein the aqueous alkali metal hydroxide having a concentration of at least 30%, which is heated to close to the boiling point, is introduced (3) at the upper end of the dividing wall (23) where the alkoxide solution is taken off (24).

13. A process as claimed in claim 11, wherein the column (22) is operated at a pressure of from 0.5 to 10 bar.

## Revendications

1. Procédé de production de méthylates alcalins à partir de lessives alcalines éventuellement mélangées à du méthanol et de méthanol, dans une colonne de réaction (4), comportant au moins 5 plateaux théoriques entre le point d'entrée de la lessive alcaline (3) et le point d'addition du méthanol (8), et décomposition du mélange de vapeurs méthanol - eau formé lors de la réaction dans une colonne de rectification (12), **caractérisé en ce que**, pour la colonne de réaction (4) configurée en colonne à plateaux à cloches, à soupapes ou à fond perforé, on choisit des plateaux dans lesquels un maximum de 5% du liquide traverse en pluie les plateaux respectifs, ou **en ce que** la colonne de réaction (4) est configurée en colonne à corps de remplissage ou à garnissage structuré, où dans toutes les zones partielles de la section transversale de la colonne, qui correspondent à plus de 2% de la section transversale totale de la colonne, la proportion moyenne du courant de liquide au courant de vapeur n'est pas dépassée de plus de 15%, **en ce que** dans la zone périphérique voisine du manteau de la colonne, qui correspond à environ 2 à 5% de la section transversale totale de la colonne, la densité de ruissellement du liquide est réduite d'une quantité allant jusqu'à 100%, et la paroi intérieure de colonne de la colonne de réaction (4), (24) équipée de corps de remplissage ou d'un gamissage structuré présente une température de 3 à 10°C supérieure à la température intérieure de la colonne de réaction, que la colonne de réaction est conduite sans reflux et que les colonnes de réaction et de rectification sont des colonnes individuelles (4) et (12) ou sont intégrées à un unique manteau de colonne (22).

2. Procédé selon la revendication 1, **caractérisé en ce que** la lessive alcaline est préparée par voie électrochimique, de préférence par un procédé à membrane.

3. Procédé selon la revendication 1 ou 2, **caractérisé en ce que** la lessive alcaline présente une concentration d'au moins 30% et que, avant son entrée dans la colonne de réaction (4), (24), elle est chauffée par un échangeur de chaleur (2) jusqu'au voisinage du point d'ébullition correspondant à la pression qui règne dans la colonne de réaction.

4. Procédé selon l'une quelconque des revendications 1 à 3, **caractérisé en ce que** la fraction massique de méthanol est de 10 à 50 fois la fraction massique de l'eau introduite avec la lessive alcaline, lorsque la lessive alcaline est de la soude caustique ou de la potasse caustique.

5. Procédé selon l'une quelconque des revendications 1 à 4, **caractérisé en ce que** le méthanol formé à partir du mélange de méthanol - eau d'une teneur en eau de 20 à 100 ppm dans la colonne de rectification (12), (25) avec une proportion de reflux d'au moins 0,5, est renvoyé dans l'extrémité inférieure de la colonne de réaction (8) via un condenseur partiel (14) et un compresseur de vapeurs (10).

6. Procédé selon la revendication 5, **caractérisé en ce que** le courant de méthanol obtenu après la rectification, d'une teneur en eau de 20 à 100 ppm, n'est envoyé dans le bas de la colonne de réaction (8) qu'en une quantité partielle de 10 à 70%, et que la quantité partielle restante est, en un courant unique ou partagée en plusieurs courants partiels (20), introduite de 1 à 10 plateaux théoriques au-dessous du point d'amenée de la lessive alcaline (3).

7. Procédé selon l'une quelconque des revendications 1 à 6, **caractérisé en ce que** l'on alimente du méthanol frais, à des températures allant jusqu'au point d'ébullition, en tête de la colonne de rectification (19).

8. Procédé selon la revendication 1, **caractérisé en ce que** les colonnes de réaction (4) et de rectification (12) sont des colonnes individuelles.

9. Procédé selon la revendication 8, **caractérisé en ce que** le méthanol formé à partir du mélange de méthanol - eau d'une teneur en eau de 20 à 100 ppm dans la colonne de rectification (12) avec compresseur de vapeurs (10) en amont avec une proportion de reflux d'au moins 0,5, est renvoyé dans l'extrémité inférieure de la colonne de réaction (4) via un condenseur partiel (14).

10. Procédé selon la revendication 8, **caractérisé en ce que** la colonne de réaction (4) est conduite à une pression de 0,5 à 40 bar.

11. Procédé selon la revendication 1, **caractérisé en ce que** les colonne de réaction et de rectification sont intégrées dans un unique manteau de colonne (22), dans lequel la zone inférieure de la colonne est partagée par une cloison de séparation verticale (23) placée entre les parois de colonne, qui empêche le mélange de courants de liquide et de vapeur dans cette zone partielle de la colonne, les deux parties de cette zone divisée longitudinalement présentant un évaporateur propre (5), (16) par où l'on soutire la solution d'alcoolate (6) ainsi que l'eau (18).

12. Procédé selon la revendication 11, **caractérisé en ce que** la lessive alcaline d'une concentration d'au moins 30% est alimentée, chauffée au voisinage du point d'ébullition, à l'extrémité supérieure (3) de la cloison de séparation (23) d'où l'on soutire la solution d'alcoolate (24).

13. Procédé selon la revendication 11, **caractérisé en ce que** la colonne (22) est conduite à une pression de 0,5 à 10 bar.
